# EUROPÄISCHE PATENTSCHRIFT

(11) **EP 3 344 281 B1**
(45) Veröffentlichungstag und Bekanntmachung des Hinweises auf die Patenterteilung: **09.12.2020**
(21) Anmeldenummer: 16759756.6
(22) Anmeldetag: 31.08.2016
(51) Int. Cl.: A61K 38/46, A61K 9/00, A23L 33/195, A61K 38/16, A23L 33/00

(54) **PHARMAZEUTISCHE ZUSAMMENSETZUNG, ENTHALTEND MINDESTENS EIN VERDAUUNGSENZYM, ZUR VERWENDUNG BEI DER KÜNSTLICHEN ERNÄHRUNG**
PHARMACEUTICAL COMPOSITION CONTAINING AT LEAST ONE DIGESTIVE ENZYME FOR USE IN ARTIFICIAL NUTRITION
COMPOSITION PHARMACEUTIQUE CONTENANT AU MOINS UNE ENZYME DIGESTIVE POUR UTILISATION DANS LE CADRE D'UNE NUTRITION ARTIFICIELLE

(30) Priorität: 04.09.2015 DE 102015114859
(43) Veröffentlichungstag der Anmeldung: 11.07.2018
(73) Patentinhaber: Nordmark Arzneimittel GmbH & Co. KG, 25436 Uetersen (DE)
(72) Erfinder: FORSSMANN, Kristin, 25436 Heidgraben (DE)
(74) Vertreter: RGTH
(86) Internationale Anmeldenummer: PCT/EP2016/070516
(87) Internationale Veröffentlichungsnummer: WO 2017/037114

(56) Entgegenhaltungen:
- WO-A1-2006/044529
- DE-A1- 10 243 208
- DE-A1-102009 006 594
- US-A1- 2014 276 632
- FERRIE S ET AL: "Pancreatic enzyme supplementation for patients receiving enteral feeds", NUTRITION IN CLINICAL PRACTICE, WILLIAMS AND WLIKINS, BALTIMORE, MD, US, Bd. 26, Nr. 3, 1. Juni 2011 (2011-06-01), Seiten 349-351, XP009178443, ISSN: 0884-5336, DOI: 10.1177/0884533611405537
- HEUBI JAMES E ET AL: "Safety and Efficacy of a Novel Microbial Lipase in Patients with Exocrine Pancreatic Insufficiency due to Cystic Fibrosis: A Randomized Controlled Clinical Trial", JOURNAL OF PEDIATRICS, Bd. 176, September 2016 (2016-09), Seite 156, XP029705650, ISSN: 0022-3476, DOI: 10.1016/J.JPEDS.2016.05.049

## Beschreibung

Die Erfindung betrifft eine pharmazeutische Zusammensetzung, enthaltend mindestens ein Verdauungsenzym.

Wenn ein Patient die notwendige Nahrung nicht peroral zu sich nehmen kann, muss dieser künstlich ernährt werden. Die natürliche Nahrungsaufnahme über den Mund kann bei einer Vielzahl von Erkrankungen behindert oder eingeschränkt sein. Beispielsweise sind dies schwere Traumata, Operationen und Bewusstlosigkeit. Zudem ist eine künstliche Ernährung erforderlich bei funktionellen Störungen des Verdauungstrakts, zum Beispiel bei massiven Kau- und Schluckbeschwerden nach einem Schlaganfall, bei Gesichtsfrakturen oder Hals-Tumoren, oder bei Erkrankungen der Verdauungsorgane wie Stenosen des Darms, Mukoviszidose, Pankreatitis oder Morbus Crohn. Weitere Indikationen sind Kachexie (starke Abmagerung) bei Krebserkrankungen, immunologischen oder chronischen Erkrankungen. Auch bei manchen Patienten mit seniler Demenz, Morbus Parkinson oder Anorexia nervosa kann es nötig sein, die Nahrung künstlich zuzuführen.

Die künstliche Ernährung kann über den Verdauungstrakt (enterale Ernährung) erfolgen, d. h. über eine Sonde. Da bei der enteralen Ernährung die Nahrung über eine Sonde in den Magen-Darm-Trakt geleitet wird, ist dies nur möglich, wenn der Verdauungstrakt noch funktioniert. Die Wahl der Sonde hängt dabei von der voraussichtlichen Ernährungsdauer, der Art der Erkrankung und dem Zustand des Patienten ab. Grundsätzlich gibt es mehrere Möglichkeiten der Sondenernährung:
Dauert es voraussichtlich weniger als vier Wochen, bis der Patient wieder selbst essen kann, ist in der Regel eine Sonde sinnvoll, die über die Nase eingeführt wird. Die Sonde wird je nach dem, wo sie endet, als Magen-Sonde (gastrale oder Nasogastral-Sonde), Nasointestinal-Sonde (die Sonde endet im Darm, wie Zwölffingerdarm oder Leerdarm), Nasoduodenal-Sonde (die Sonde endet im Zwölffingerdarm oder Duodenum) oder Nasojejunal-Sonde (die Sonde endet im Leerdarm oder Jejunum) bezeichnet.

Die Sonde stellt in diesen Fällen einen dünnen Schlauch dar, der durch Nase oder Mund, Rachen und Speiseröhre in den Magen oder bis in einen Teil des Dünndarms führt. Die Verwendung einer Sonde setzt eine ungehinderte Passage in Nase und Rachen voraus. Bei Sonden, die im Magen enden, muss zudem die Magenentleerung funktionieren. Diese Form der Ernährung nutzt den gesamten Verdauungstrakt und entspricht damit der normalen Ernährung. Die Nahrung wird vom Magen portionsweise an den Darm weitergeleitet, und die gastrointestinale Hormonproduktion wird auf diese Weise aktiviert. Eine Alternative zur nasogastralen Sonde, wenn beispielsweise die Magenentleerung gestört ist, stellt die sogenannte Nasojejunal-Sonde dar, wobei die Sonde bis in den Dünndarm geleitet wird. Sie führt über die Nase durch den Magen bis in einen bestimmten Abschnitt des Dünndarms, das Jejunum. Diese Sonde wird in der Regel mithilfe eines Endoskops eingeführt. Weil diese Sonde dünner als eine nasogastrale Sonde ist, stört sie den Patienten weniger und kann deshalb meist länger verbleiben. Die Gabe der Nahrung über die Nasojejunal-Sonde erfolgt in der Regel kontinuierlich, weil das Jejunum keine derartige Speicherfunktion wie der Magen aufweist und deshalb größere Nahrungsportionen weniger gut verträgt.

Wenn die enterale Ernährung voraussichtlich länger als drei Wochen beibehalten werden muss, ist es sinnvoll, eine perkutane Sonde durch die Bauchdecke in den Magen zu legen, eine sog. PEG-Sonde (PEG: perkutane endoskopische Gastrostomie). Dabei wird unter örtlicher Betäubung im Rahmen einer Magenspiegelung ein dünner Kunststoffschlauch durch die Bauchdecke in den Magen geführt und gegen Verrutschen gesichert. In manchen Fällen wird die Sonde auch in den angrenzenden Dünndarm gelegt, eine sog. PEJ-Sonde (PEJ: perkutane endoskopische Jejunostomie). Der Eingriff dauert wenige Minuten, ist weitgehend schmerzfrei und gestattet auch zusätzliches Essen und Trinken sowie Schluck- und Esstraining.

Die Verabreichung der Sondennahrung erfolgt ähnlich wie bei einer Infusion, mittels Schwerkraft oder elektronisch geregelter Pumpe, meist kontinuierlich, seltener in Portionen als sogenannte "Bolusgabe". Zur Vermeidung von Erbrechen sitzt der Patient dabei, oder der Oberkörper wird hoch gelagert. Bevorzugt ist die Applikation der Nahrungssubstrate mit Hilfe einer Ernährungspumpe, bei der die Tropfgeschwindigkeit exakt eingestellt und somit die gewünschte Substratmenge genau zugeführt werden kann. Eine ungenaue und unregelmäßige Zufuhr kann beim Betroffenen zu gastrointestinalen Unverträglichkeiten führen. Die Zufuhrrate des Nahrungssubstrates liegt beispielsweise bei 120 ml/h - 150 ml/h.

Jeder Mensch hat dabei seinen individuellen Bedarf an Nahrung und Flüssigkeit, der von verschiedenen Faktoren abhängt, wie Alter, Größe und Gewicht, Gesundheitszustand usw. Der behandelnde Arzt entscheidet unter Abwägung aller Aspekte über Art und Menge der zu verabreichenden Sondennahrung sowie der Flüssigkeitszufuhr. Vor Beginn einer künstlichen Ernährung wird daher zunächst der Ernährungszustand des Patienten ermittelt, um die Energie- und Nährstoffzufuhr in geeigneter Weise dosieren zu können. Zur Abschätzung der benötigten Energiezufuhr wird beispielweise der Ruheenergieverbrauch anhand Körpergröße, Gewicht, Alter und Geschlecht berechnet; bei Erwachsenen liegt dieser normalerweise bei etwa 20 bis 24 kcal pro kg Körpergewicht pro Tag.

Bei der Sondennahrung handelt es sich um Nährlösungen, welche die notwendigen Nährstoffe in ausgewogener Zusammensetzung enthalten. Die Nährstofflösungen enthalten die notwendigen Eiweiße, Kohlenhydrate, Fette, Vitamine, Mineralstoffe und Spurenelemente. Auch Medikamente können mit/in der Nährstofflösung verabreicht werden. Zusätzlich kann Flüssigkeit in Form von Wasser oder Tee durch die Sonde gegeben werden.

### Stand der Technik

Es steht eine Vielzahl an Sondennahrung zur Verfügung: die beispielsweise in Klinik- oder Diätküchen selbst hergestellte Sondenkost, nährstoffdefinierte oder hochmolekulare Diäten sowie chemisch definierte oder niedermolekulare Diäten, einschließlich Sonderformen, die auf spezielle Erkrankungen zugeschnitten sind. Industriell bzw. synthetisch hergestellte Produkte sind der selbst gemachten Kost stets vorzuziehen, da sie hygienisch sicherer sind und auch über längere Zeit eine Bedarfsdeckung an allen Nährstoffen bieten. Zudem können nicht alle Lebensmittel sondengängig gemacht werden. Auch kann eine falsche Dosierung der Nährstoffe in den Nährlösungen zu Komplikationen führen. Zudem sind industriell hergestellte Produkte "bilanziert", d. h. diese sind genau auf den Nährstoffbedarf abgestimmt.

Die synthetisch hergestellten nährstoffdefinierten oder hochmolekularen Diäten enthalten alle Nährstoffe - wie bei herkömmlicher Ernährung - in hochmolekularer Form: die enthaltenen Proteine sind beispielsweise die üblichen intakten Proteine, Fette sind langkettige Triglyceride und Kohlenhydrate sind Poly-, Oligo- oder Monosaccharide. Die Polymere müssen erst enzymatisch aufgespalten werden, bevor sie im Darm resorbiert werden können, so dass eine intakte Verdauungsleistung für diese Sondenkost notwendig ist. Die Nährlösungen sind regelmäßig so eingestellt, dass diese zwischen 1 und 1,5 kcal pro ml liefern, und sind mit und ohne Ballaststoffe erhältlich.

Für Patienten mit eingeschränkter Verdauungsleistung sind die chemisch definierten oder niedermolekularen Diäten geeignet, die die Nährstoffe in Form von schnell verwertbaren Monomeren, wie Monosacchariden und einzelnen Aminosäuren, enthalten, die ohne enzymatische Aufspaltung im Dünndarm aufgenommen werden können. So können bei einer Fettverdauungsstörung beispielsweise Produkte mit mittelkettigen Triglyceriden eingesetzt werden. Bei Kurzdarmsyndrom und Malabsorption oder zur Ruhigstellung von hinteren Darmabschnitten sind ballaststofffreie Diäten mit kurzkettigen Peptiden empfehlenswert.

Sondenkost für Patienten gibt es in Glasflaschen oder Kunststoffbeuteln. Diese werden mit einem speziellen Schlauchstück direkt mit der Sonde verbunden. Bettlägerige Patienten erhalten ihre Tagesration meist kontinuierlich, mit einer Pause von etwa sechs Stunden während der Nacht. Eine elektronische Pumpe kann die Zufuhrgeschwindigkeit kontrollieren. Mobile Patienten können die Nahrung auch portionsweise aufnehmen, ähnlich dem üblichen Essrhythmus. Beispielsweise enthält eine Tagesration von 1500 bis 2000 ml alle nötigen Nährstoffe, Vitamine und Mineralstoffe. Nur Flüssigkeit muss noch ergänzt werden.

Aus dem Stand der Technik ist die Sondenernährung bekannt und zahlreiche Anwendungen werden beschrieben:
So bezieht sich das ukrainische Gebrauchsmuster UA 10025 U auf ein Verfahren, um in der frühen postoperativen Phase Magen-Darm-Bluten bei Patienten mit enteraler Ernährung zu verhindern. Hierbei wird die Säureproduktion im Magen durch Verabreichung einer Acidin-Pepsin-Lösung in den Dünndarm während des Zeitraums der künstlichen Ernährung verringert. Pepsin ist ein Verdauungsenzym, eine sog. Peptidase, das im Magen von Wirbeltieren und damit auch vom Menschen gebildet wird. Es ist ein Phosphoprotein mit 327 Aminosäuren. Acidin ist ein Betain-Hydrochlorid; dies ist ein Oxidationsprodukt des Cholins und ein Derivat der Aminosäure Glycin. Kommerziell verfügbares Pepsin stammt meistens aus dem Magen des Schweins und bringt daher die Nachteile tierischer Produkte mit sich, wie Risiken der Kontamination und Verträglichkeitsprobleme. Die Verwendung einer Lipase wird hier nicht erwähnt.

Ferner offenbart die DE 10 2009 006 594 A1 pharmazeutische Präparate zur Behandlung von Pankreasinsuffizienz, beispielsweise Mukoviszidose oder andere Pankreaserkrankungen. Als pharmazeutisches Präparat wird beispielsweise eine flüssige Verabreichungsform beschrieben, wobei bevorzugt eine Bakterien-Lipase von der Art Burkholderia, Pseudomonas oder Burkholderia plantarii eingesetzt wird. Auch die Anwendung von künstlicher Ernährung wird erwähnt. Die erfindungsgemäße Lehre wird jedoch nicht beschrieben.

Gemäß Rosenfeld, M. et al.: Nutritional effects of long-term gastrostomy feedings in children with cystic fibrosis; J. Am. Diet. Assoc., 1999, Bd., 99, S. 191-194 werden ernährungsphysiologische Wirkungen bei der Langzeit-Gastrostomieernährung von Kindern mit zystischer Fibrose untersucht. Die Patienten litten alle unter Pankreasinsuffizienz und ihnen wurden Pankreasenzym-Zusätze verabreicht. Die Nahrungsaufnahme erfolgte über eine kontinuierliche Infusion während der Nacht (s. S. 193, linke Spalte, 2. Absatz). Diese Vorgehensweise ist mit einer Reihe an Nachteilen verbunden: Der Patient hat keine Ruhe während der Nacht und kann sich daher nicht so schnell erholen. Die Pankreasenzym-Zusätze wurden nämlich vor oder nach erfolgter Nahrungsaufnahme in der Nacht oder am Morgen verabreicht, was die Nachtruhe jeweils unterbricht bzw. vorzeitig beendet. Zudem liegt damit die Nahrung nicht gleichzeitig zusammen mit den Enzymen-Zusätzen im Magen-Darm-Trakt vor. Die Verdauungsprobleme werden hierdurch nicht in erwünschter Weise beseitigt. Weiterhin sind die Pankreasenzyme wahrscheinlich tierischen Ursprungs, d. h. diese stammen vom Schwein, so dass ein Kontaminationsrisiko mit Krankheitserregern besteht. Zudem liegt die bekannte Problematik vor, dass eine mangelnde Aktivität der Schweine-Lipase während des Magendurchgangs vorliegt, da diese nur bei pH 5 bis 9 aktiv ist. Eine ausreichende Wirksamkeit der Enzym-Zusätze ist daher nicht gewährleistet.

In der US 2010/0239559 A1 wird eine Ernährungsprodukt-Zusammensetzung beschrieben, umfassend ein Enzym, ausgewählt aus der Gruppe, bestehend aus Lipase, einer Amylase, Protease und Kombinationen hiervon, wobei das Enzym formuliert wurde, um in wässerigem Medium besonders stabil zu sein, sowie ein Nahrungsergänzungsmittel. Unter zahlreichen Möglichkeiten wird auch eine Bakterien-Lipase, wie Pseudomonas cepacia-Lipase, beschrieben. Zudem wird als mögliche Verabreichungsform auch eine Magensonde erwähnt. Damit die Enzyme in einer stabilisierten Form bereitgestellt werden können, um eine dauerhafte Stabilität in wässerigem Medium zu ermöglichen, werden die Enzyme in Form von Kristallen hergestellt und gegebenenfalls von Polymeren umhüllt. Es spielen daher andere Aspekte als in der vorliegenden Erfindung eine Rolle und andere Schwerpunkte liegen vor.

Weiterhin sind aus dem Stand der Technik zahlreiche Veröffentlichungen bekannt, die den Aufbau von mehrlumigen Sonden beschreiben (s. beispielsweise DE 102 43 208 B4 und US 5 527 280 A).

In einigen Fällen kann die Sondenernährung jedoch zu Problemen führen: Beispielsweise kann Durchfall auftreten. Dies kann darauf zurückgehen, dass die Nahrung zu schnell gegeben wurde oder zu kalt war - die Sondennahrung sollte stets Zimmertemperatur haben. Auch eine Unverträglichkeit gegenüber einem Bestandteil der Sondennahrung ist möglich, oder aber die Verdauung des Patienten verursacht Komplikationen. Es ist daher zweckmäßig, die Verdauung bei Patienten mit künstlicher Ernährung zu unterstützen.

Um die Verdauung bei der künstlichen Ernährung zu verbessern, ist es beispielsweise gebräuchlich, bei der Sondenernährung Verdauungsenzyme in Form fester, magensaftresistent überzogener, multipartikulärer Pankreasenzymzubereitungen peroral oder über großlumige Sonden (z.B. 16 bis 18 French (= Fr); 1 Fr = 0,33 mm äußerer Durchmesser) bolusweise zu geben. Dies wird beispielsweise in Shlieout et al. "Administration of CREON® Pancrelipase Pellets via Gastrostomy Tube is Feasible with No Loss of Gastric Resistance or Lipase Activity", Journal of Clinical Investigation, 2011, S. 1 bis 7, im Einzelnen beschrieben. Jedoch ist dieses Vorgehen eher unphysiologisch und weicht völlig von den normalen Essgewohnheiten beim gesunden Menschen ab. Zudem kann hierbei keine optimale Durchmischung der Verdauungsenzyme mit der Sondennahrung garantiert werden, so dass die Wirkung in jedem Fall nur suboptimal und ggf. unzureichend ist und häufig keine deutliche Verbesserung der Verdauung resultiert. Zusätzlich ist dieses Vorgehen in der praktischen Umsetzung mit zusätzlichen Belastungen für die Patienten (z. B. nächtliches Wecken) verbunden.

Die US 2014/276632 A1 betrifft eine Zusammensetzung für die künstliche Ernährung enthaltend Verdauungsenzyme, die über eine Magensonde verabreicht werden. Beispielhaft wird Burkholderia Lipase genannt.

Aus der WO 2006/044529 A1 ist eine Zusammensetzung bekannt, die Burkholderia Lipase zur Behandlung von Pankreasinsuffizienz bei Bukholderia Lipase bei cystischer Fibrose vorschlägt.

### Darstellung der Erfindung: Aufgabe, Lösung, Vorteile

Der vorliegenden Erfindung liegt daher die Aufgabe zugrunde, die bekannten Nachteile aus dem Stand der Technik zu vermeiden. Insbesondere soll es möglich sein, die Verdauung bei der künstlichen Ernährung zuverlässig zu verbessern, wobei jedoch zusätzliche Belastungen des Patienten weitgehend vermieden werden sollen.

Die geschilderte Aufgabe wird gelöst durch eine pharmazeutischen Zusammensetzung zur Verwendung bei der Behandlung von Verdauungsstörungen, wobei die pharmazeutische Zusammensetzung in Kombination mit einer für die künstliche Ernährung vorgesehenen Sondennahrung in der Behandlung von Verdauungsstörungen bei der künstlichen Ernährung verwendet wird, wobei die pharmazeutische Zusammensetzung mindestens ein Verdauungsenzym, bevorzugt eine Lipase, besonders bevorzugt eine bakterielle Lipase, ganz besonders bevorzugt Burlulipase (INN) in flüssiger Darreichungsform enthält oder hieraus besteht, wobei die pharmazeutische Zusammensetzung und die Sondennahrung gleichzeitig, aber separat voneinander verabreicht werden.

Bevorzugterweise sind die pharmazeutische Zusammensetzung und die Sondennahrung derart formuliert, dass beide gleichzeitig verabreicht werden können, aber ohne vorab miteinander in Kontakt zu kommen. Außerdem hat es sich als vorteilhaft erwiesen, wenn die pharmazeutische Zusammensetzung und die Sondennahrung derart formuliert sind, dass eine kontinuierliche Verabreichung möglich ist.

Die Erfindung bezieht sich daher auf die Verwendung einer pharmazeutischen Zusammensetzung mit einer für die künstliche Ernährung vorgesehenen Sondennahrung zur Herstellung eines Kombinationsproduktes in der Behandlung von Verdauungsstörungen bei der künstlichen Ernährung, wobei die pharmazeutische Zusammensetzung mindestens ein Verdauungsenzym, bevorzugt eine Lipase, besonders bevorzugt eine bakterielle Lipase, ganz besonders bevorzugt Burlulipase (INN) in flüssiger Darreichungsform enthält oder hieraus besteht, wobei das Kombinationsprodukt zur Verabreichung derart hergerichtet ist, dass die pharmazeutische Zusammensetzung und die Sondennahrung für eine gleichzeitige, aber separat voneinander erfolgende Verabreichung formuliert sind.

Die vorliegende Erfindung ermöglicht daher die Bereitstellung für eine parallele, kontinuierliche Applikation einer flüssigen Darreichungsform einer pharmazeutischen Zusammensetzung, enthaltend mindestens ein Verdauungsenzym, und der Sondennahrung. Durch die gleichzeitige, kontinuierliche, aber separate Gabe gelingt in der Regel eine Ausschöpfung der vollen Verdauungsleistung des Patienten.

Als "Verdauungsenzyme" werden hierbei üblicherweise Enzyme bezeichnet, die bevorzugt den drei Enzymklassen entstammen, die für die Verdauung der drei Grundbestandteile der Nahrung erforderlich sind - Lipasen für Fette, Amylasen für Kohlenhydrate, Proteasen für Proteine. Als solche sind diese beim Gesunden in ausreichender Menge im exokrinen Bauchspeicheldrüsensekret enthalten.

Die derzeit übliche Behandlung einer auf Enzyminsuffizienz basierenden unzureichenden Verdauungsleistung besteht in der Verabreichung von Präparaten, die einen Extrakt aus Schweinepankreas - und damit Verdauungsenzyme aus den drei vorgenannten Enzymklassen - enthalten. Dieser Wirkstoff "Pankreatin" ist im Europäischen Arzneibuch (Ph. Eur.) als "Pankreas-Pulver" (Pancreas Powder), im US-amerikanischen Arzneibuch (USP) als "Pancreatin" bzw. "Pancrelipase" monographiert.

Im Rahmen der vorliegenden Erfindung ist es ganz besonders bevorzugt, wenn als das enthaltene mindestens eine Verdauungsenzym die bakterielle Lipase "Burlulipase" (INN) eingesetzt wird. Bekanntermaßen produzieren Bakterien eine Vielzahl lipolytisch aktiver Enzyme. In der US 5 645 832 oder der US 5 489 530 wurde bereits eine aus Bakterien gewonnene Lipase im Einzelnen beschrieben. Burlulipase ist eine Triacylglycerolacylhydrolase (EC 3.1.1.3), die eine mit den durch *Burkholderia plantarii* und *Burkholderia glumae* produzierten Lipasen übereinstimmende Aminosäuresequenz aufweist. Burlulipase kann daher insbesondere hergestellt werden durch einen klassischen Fermentationsprozess, in dem *Burkholderia plantarii,* ein nicht-rekombinantes, gram-negatives Bakterium als Produktionsstamm eingesetzt wird.

Burlulipase zeichnet sich insbesondere durch seine gute Säurestabilität in Verbindung mit einer hohen spezifischen Aktivität aus, die auch bei den sauren pH-Werten im Magen weitgehend erhalten bleibt. Demgegenüber zeigt beispielsweise Schweine-Lipase bei einem pH-Wert im Bereich von 2,0 bis 4,0 nach 2 Stunden bereits eine um 85% verringerte Aktivität. Außerdem kann Burlulipase in vorteilhafter Weise als flüssige Formulierung zur Verfügung gestellt werden, ohne dass ein Aktivitätsverlust beobachtet wird. Es ist daher besonders vorteilhaft, dass für die künstliche Ernährung eine geeignete flüssige Formulierung verwendet werden kann und nicht auf feste Verabreichungsformen, wie beispielsweise multipartikuläre Formen, zurückgegriffen werden muss.

Reine Burlulipase kann eine spezifische Aktivität von mehr als 3.500 U/mg (Tributyrin-Einheiten pro Milligramm Protein) aufweisen. Aufgrund dieser hohen lipolytischen Aktivität ist Burlulipase zur Unterstützung der Verdauungsleistung bei gesunden und kranken Menschen besonders geeignet. Die Lipase-Konzentration von Burlulipase ist sehr hoch, so dass nur geringe Substanzmengen, d. h. eine geringere Masse bzw. ein geringeres Volumen an Lösung, verabreicht werden müssen. Der pH-spezifische Aktivitätsbereich von Burlulipase liegt zwischen 4 und 9, wobei das Molekül im gesamten pH-spezifischen Aktivitätsbereich stabil ist und erst bei pH-Werten unterhalb von 3 bereits nach kurzer Zeit zunehmend inaktiviert wird. Damit überwindet Burlulipase als Wirkstoff die Einschränkungen hinsichtlich Aktivität und Stabilität von Pankreatin aus Schweine-Pankreas. Der pH-spezifische Aktivitätsbereich der Pankreas-Lipase liegt zwischen 5 und 9 bei deutlicher, säurebedingter Inaktivierung bereits unterhalb von pH 4. Dies bedeutet, dass mittels Burlulipase eine lipolytische Wirkung im Magen-Darm-Trakt in mehrfacher Hinsicht deutlich effektiver zu erzielen ist, als mit den für die Therapie von Maldigestion auf dem Markt bislang erhältlichen Produkten.

Burlulipase ist also in einem weiten pH-Bereich enzymatisch aktiv und beständig sowie in geeigneter wässriger Lösung und Verpackung über mehrere Monate lagerstabil.

Bevorzugt ist, dass in der pharmazeutischen Zusammensetzung als Verdauungsenzym eine bakterielle Lipase in Form von Burlulipase vorliegt. Die pharmazeutische Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase (INN) in flüssiger Darreichungsform, ist vorzugsweise eine flüssige Darreichungsform von Verdauungsenzymen. Hierbei handelt es sich beispielsweise um eine Lösung, eine Suspension oder eine Emulsion. Handelt es sich bei der vorgenannten pharmazeutischen Zusammensetzung um eine feste oder halbfeste Arzneiform von Verdauungsenzymen, so lässt sich diese durch Auflösen oder Dispergieren in einem physiologisch verträglichen, meist Wasser-basierten Lösungsmittel bzw. Dispersionsmedium vorzugsweise leicht in eine solche flüssige Darreichungsform überführen.

Es hat sich als besonders vorteilhaft herausgestellt, wenn die künstliche Ernährung bei gleichzeitiger Gabe der pharmazeutischen Zusammensetzung mittels eines zumindest doppellumigen Sondensystems durchgeführt wird, wobei die Sondennahrung in einem Lumen und die pharmazeutische Zusammensetzung in flüssiger Darreichungsform im anderen Lumen verabreicht wird.

Dies hat den Vorteil, dass die pharmazeutische Zusammensetzung nicht bereits vorher mit der Sondennahrung in Kontakt kommt und so das oder die enthaltenen Enzyme bereits aktiviert werden und mit der Verdauung der Nahrung beginnen. Durch die parallele und kontinuierliche Verabreichung kommt die pharmazeutische Zusammensetzung erst im Magen oder Darm in Kontakt mit der Nahrung, so dass erst dort die Verdauung beginnt und unterstützt wird. Hierdurch gelingt es, die Verdauung eines Patienten, der künstlich ernährt wird, deutlich zu verbessern. Insbesondere mit einer entsprechend verdünnten Burlulipase-Lösung wurden gute Ergebnisse erzielt.

Die parallele und kontinuierliche Verabreichung von pharmazeutischer Zusammensetzung in Form einer flüssigen Darreichungsform und Sondennahrung gelingt besonders vorteilhaft über eine zumindest doppellumige Sonde. Als Lumen bezeichnet man den inneren Hohlraum des Schlauches der Sonde. Der Schlauch einer Sonde kann generell aus einem oder aus mehreren Lumen bestehen. Eine Magensonde mit nur einem Lumen ist somit ein einfacher Schlauch, der aufgebaut ist, wie ein einfaches, biegsames Röhrchen. Ein typisches Beispiel für eine Magensonde mit nur einem Lumen ist eine Ableitungssonde, die zum Ablaufen von Magenflüssigkeit aus dem Magen nach außen dienen kann.

Eine doppellumige Sonde ist daher eine doppelläufige Sonde, die zwei Lumen aufweist z. B. eine doppelläufige Magen-Darm-Sonde, namentlich Lagerlöf-Sonde, wie sie für die Pankreasfunktionsdiagnostik zum getrennten Absaugen von Magensekret und Duodenalinhalt eingesetzt wird. Die anderen Eigenschaften des Sondensystems bleiben hiervon unberührt und können entsprechend ausgewählt werden. Beispielsweise kann die Sonde je nach Länge im Magen (gastral), im Zwölffingerdarm (duodenal), oder im Leerdarm (jejunal) enden. Bevorzugt können Sonden mit mehr Lumen (mehrlumige Sonden) verwendet werden, besonders bevorzugt sind Sonden mit 2 oder 3 Lumen

Die Art und das Material der Sonde sind nicht besonders beschränkt. Meist wird als physiologisch inertes und daher verträgliches Material für die Sonde Polyvinylchlorid, Polyurethan oder Silikon bzw. Silikonkautschuk verwendet, wobei alle Sonden im Allgemeinen so ausgeführt sind, dass sie röntgenologisch darstellbar sind. Das weiche Material ist speiseröhren- und schleimhautfreundlich. Sonden können je nach ihrer Verwendung eine Länge von beispielsweise 100 bis 130 cm aufweisen. Die gebräuchlichsten Größen sind: CH 6 bis 8 für Neugeborene, CH 8 bis 10 für Kleinkinder, CH 10 bis 12 für Kinder, CH 12 bis 18 für Erwachsene. Ein Charrière oder CH (auch Charr oder French (= Fr)) bezeichnet in der Medizin ein Maß für den äußeren Umfang von Kanülen und Kathetern/Sonden und entspricht einem Millimeter äußeren Umfangs korrespondierend mit etwa ⅓ mm Außendurchmesser.

Der als Sonde verwendete Schlauch ist daher nur wenige Millimeter dick, so dass die Sondennahrung absolut frei von größeren Partikeln sein sollte - bereits geronnenes Eiweiß oder auskristallisierende Mineralsalze könnten den Schlauch verstopfen. Damit die dünne Sonde nicht verstopft, ist daher eine sorgfältige Pflege durch regelmäßiges Spülen erforderlich.

Es versteht sich daher, dass die pharmazeutische Zusammensetzung gemäß der vorliegenden Erfindung derart bereitgestellt wird, dass sie durch eine Sonde verabreicht werden kann. Die Kriterien, die hierfür erfüllt werden müssen, sind dem Fachmann im Stand der Technik bekannt.

Die Verwendung einer pharmazeutischen Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase (INN) in flüssiger Darreichungsform, bei der Sondenernährung mit Hilfe einer zumindest doppellumigen Sonde bewirkt, dass die Verdauungsenzym-haltige, flüssige Darreichungsform und die Sondennahrung getrennt voneinander verabreicht werden. Die Verdauungsenzym-haltige, flüssige Darreichungsform und die Sondennahrung werden erst im Magen-Darm-System zusammengeführt. Hierfür kann eine dünnere Sonde im Vergleich zu Sonden aus dem Stand der Technik verwendet werden, mit denen beispielsweise zusätzlich Arzneimittel zur Verbesserung der Verdauung an den Patienten verabreicht werden. Durch die zusätzliche Verabreichung der pharmazeutischen Zusammensetzung in flüssiger Darreichungsform entstehen keine Probleme bei der Verwendung des Sondensystems: Es tritt kein unerwünschter Rückfluss und keine Verstopfung der Sonde auf, da die pharmazeutische Zusammensetzung in ihrer flüssigen Darreichungsform ohne Probleme sondengängig ist. Somit entfällt auch ein vorzeitiger oder zusätzlicher Austausch der Sonde, bedingt durch Rückfluss bzw. Verstopfung aufgrund schwierig zu verabreichender Arzneiformen, wie Pellets.

Ein weiterer Vorteil der Erfindung ist, dass die Verdauungsenzym-haltige pharmazeutische Zusammensetzung nicht mehr direkt in die Sondennahrung gegeben werden muss. Weiterhin ist es auch nicht erforderlich, den Patienten während der Nacht alle 3 bis 4 Stunden, beispielsweise für eine Enzymaufnahme, zu wecken. Vielmehr kann die Verdauungsenzym-haltige pharmazeutische Zusammensetzung in ihrer flüssigen Darreichungsform parallel zur Sondennahrung verabreicht werden, so dass der Patient trotz künstlicher Ernährung durchschlafen kann. Hierdurch wird der Patient deutlich weniger belastet und kann schneller genesen. Auch im Falle von bewusstlosen bzw. komatösen und beatmeten Patienten stellt dies eine erhebliche Entlastung sowohl für den Patienten, als auch für das Pflegepersonal dar.

Gemäß einer bevorzugten Ausführungsform der Erfindung wird die pharmazeutische Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase (INN) in flüssiger Darreichungsform, und die Sondennahrung jeweils von einem separaten Behältnis dem jeweiligen Lumen des zumindest doppellumigen Sondensystems zugeführt. Alternativ können die pharmazeutische Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase (INN) in flüssiger Darreichungsform, und die Sondennahrung auch in einem Mehrkammerbehältnis in getrennten Kammern bereitgestellt werden und durch getrennte Zuleitungen zum jeweiligen Lumen des zumindest doppellumigen Sondensystems zugeführt werden.

Vorteilhafterweise ist die Geschwindigkeit (Volumen pro Zeit) der Verabreichung der pharmazeutischen Zusammensetzung und der Sondennahrung dann durch getrennte, separat regelbare Infusions- oder Pumpsysteme für jedes Lumen des zumindest doppellumigen Sondensystems einzeln steuerbar.

Bei der Verwendung der Verdauungsenzym-haltigen pharmazeutischen Zusammensetzung ist es von Vorteil, dass die Patienten gemäß einer bevorzugten Ausführungsform unter paralleler, kontinuierlicher Verabreichung von Verdauungsenzymen physiologisch verträglicher und unter optimaler Ausnutzung des Potentials der Medikation ernährt werden können. Diese Art der zusätzlichen Verabreichung von die Verdauung unterstützenden Arzneimitteln ist der normalen Ernährung sehr ähnlich und daher für den Patienten mit weniger Stress verbunden.

Die Verwendung einer zumindest zweilumigen Sonde bewirkt, dass die Sondennahrung erst nach Austritt aus dem Lumen mit dem/den Verdauungsenzymen in Kontakt kommt. So kann eine unerwünschte Vorverdauung der Nahrung ausgeschlossen werden. Des Weiteren ermöglicht die Verwendung der Verdauungsenzym-haltigen pharmazeutischen Zusammensetzung auch die Verabreichung durch eine Nasensonde. Dies hat den Vorteil, dass eine englumige Sonde verwendet werden kann, da die Verdauungsenzym-haltige pharmazeutische Zusammensetzung in flüssiger Darreichungsform -ganz besonders bevorzugt in Form einer Burlulipase-Lösung, vorliegt.

Bevorzugt liegt das Sondensystem in Form einer Sonde vor, die im Magen, im Zwölffingerdarm oder im Leerdarm endet. Des Weiteren ist bevorzugt, dass das Sondensystem in Form einer Sonde vorliegt, die eine Nasogastral-Sonde, Nasointestinal-Sonde, Nasoduodenal-Sonde, Nasojejunal-Sonde, PEJ-Sonde oder eine PEG-Sonde darstellt und/oder dass das Sondensystem zwei- oder mehrlumig ausgeführt ist.

In der praktischen Umsetzung der vorliegenden Erfindung können daher die sonst üblichen zusätzlichen Belastungen der Patienten, d. h. Wecken zur Verabreichung von die Verdauung unterstützenden Arzneimitteln während der Nacht, vermieden werden. Die Verabreichung der die Verdauung unterstützenden pharmazeutischen Zusammensetzung erfolgt stets zusammen mit der Sondennahrung, wodurch der Patient nicht zusätzlich für die Verabreichung der Medikation belästigt werden muss.

Weiterhin wurde festgestellt, dass die Verwendung einer bakteriellen Lipase, insbesondere Burlulipase, als das in der pharmazeutischen Zusammensetzung enthaltene mindestens eine Verdauungsenzym besondere Vorteile in der vorliegenden Erfindung bietet. So führt die zusätzliche Verabreichung von bakterieller Lipase, insbesondere Burlulipase, dazu, dass der Anteil an Lipasen deutlich erhöht und damit die Fettverdauung signifikant gesteigert wird. Die Fettverdauung ist bei Patienten mit Verdauungsschwäche i. d. R. von besonderer Relevanz. Dies führt insgesamt zu einer besseren Verträglichkeit der Nahrung beim Patienten.

Die eingesetzte bakterielle Lipase, vorzugsweise Burlulipase, wird bevorzugt nicht chemisch modifiziert, so dass ein natürliches Produkt zum Einsatz kommt, das keinen weiteren chemischen Modifikationen oder Behandlungen unterzogen wurde. Zusätzlich ist die bakterielle Lipase ein Produkt, das nicht vom Tier stammt, so dass die Gefahr möglicher, auf tierische Quellen stammender zurückzuführender Erkrankungen, wie beispielsweise Virus-Erkrankungen, generell ausgeschlossen wird.

Von Vorteil ist auch, dass die Verabreichung von Burlulipase offenbar eine gut verträgliche, nebenwirkungsarme therapeutische Option darstellt.

Zudem wird der pH-Wert des Magens oder Darms durch eine bakterielle Lipase, insbesondere Burlulipase, nicht verändert, so dass hierdurch ebenfalls keine unerwünschten Nebenwirkungen beim Patienten hervorgerufen werden und keine zusätzlichen Arzneimittel zur Kontrolle oder Einstellung des pH-Werts des Magen-/Darm-Bereichs eingenommen werden müssen.

Die Verdauungsenzym-haltige pharmazeutische Zusammensetzung wird in flüssiger Darreichungsform verabreicht, wodurch eine einfache Handhabung durch das Pflegepersonal und eine effektive Verabreichung an den Patienten resultieren. Eine flüssige Darreichungsform bietet die Möglichkeit einer bequemen und exakten Dosierung bei optimalen Voraussetzungen für eine im Magen erfolgende homogene Verteilung des/der enthaltenen Verdauungsenzyme in der (Sonden-)Nahrung.

Bei der flüssigen Darreichungsform kann es sich beispielsweise handeln um: Lösungen, Suspensionen oder Emulsionen. Eine Suspension bezeichnet dabei ein Zweiphasensystem, das aus fester innerer Phase, z. B. dem ungelösten Wirkstoff Burlulipase, besteht, die in einer Flüssigkeit dispergiert ist.

Flüssige Verabreichungsformen können entweder dadurch zubereitet werden, dass das in der pharmazeutischen Zusammensetzung enthaltene mindestens eine Verdauungsenzym direkt als flüssige Arzneiform formuliert und diese dann ggf. noch verwendungsgerecht verdünnt wird, durch Lösen der Verdauungsenzym-haltigen pharmazeutischen Zusammensetzung in einem wässrigen oder nicht-wässrigen Lösungsmittel oder durch Suspendieren bzw. Emulgieren der Verdauungsenzym-haltigen pharmazeutischen Zusammensetzung in einem geeigneten Dispersionsmedium. Als flüssige Verabreichungsform für das in der pharmazeutische Zusammensetzung enthaltene mindestens eine Verdauungsenzym, bevorzugt eine Lipase, besonders bevorzugt eine bakterielle Lipase, ganz besonders bevorzugt Burlulipase (INN), kommt demzufolge grundsätzlich jegliche bekannte flüssige Arzneiform in Betracht. Beispielsweise genannt seien: Lösungen, auch Infusionslösungen und Injektionslösungen, Suspensionen, Emulsionen, Öle, Säfte, Sirupe, Tinkturen, Tropfen, Wässer, aromatische Wässer, wässrige Säuren, Tees und dergleichen.

Insbesondere sind flüssige Verabreichungsformen, wie Lösungen, Suspensionen und Emulsionen, vorteilhaft, da deren Konzentration bei Bedarf noch durch einfach zu handhabende Verdünnung exakt und verwendungsgerecht eingestellt werden kann und sodann eine fein abgestufte Dosierung ermöglicht - ggf könnten sogar einzelne Tropfen verabreicht werden.

Burlulipase kann in der flüssigen Verabreichungsform bevorzugt in einer Konzentration von 0,0002 mg/ml bis 50 mg/ml bezogen auf das Burlulipase-Protein vorliegen.

Die pharmazeutische Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase (INN) in flüssiger Darreichungsform, kann ein oder mehrere Lösungsmittel und/oder einen oder mehrere Hilfsstoffe aufweisen. Die Lösungsmittel und/oder Hilfsstoffe sind dann vorzugsweise ausgewählt aus
- Wasser
- Salzen
- organischen Säuren
- Aminosäuren
- Detergentien
- Zuckern
- Ölen
- Viskositätsregulierungsmitteln.

Überraschenderweise ist es ausreichend, wenn die pharmazeutische Zusammensetzung nur ein Verdauungsenzym, im vorliegenden Fall eine Lipase, insbesondere Burlulipase, aufweist, um entsprechende Verdauungsstörungen wirksam zu behandeln. Andere Enzyme, wie Proteasen oder Amylasen müssen dann nicht notwendigerweise vorhanden sein.

Die zusätzliche Verabreichung einer Lipase, insbesondere von Burlulipase, im Rahmen einer künstlichen Ernährung erhöht die Menge an verfügbaren Lipasen für die Fettverdauung, welche für Menschen mit Verdauungsproblemen i. d. R. entscheidend ist.

Durch die vorliegende Erfindung wird somit eine Optimierung der Verdauungsleistung (Fettverdauung und -resorption) bei einer künstlichen Ernährung durch Steigerung der Gesamt-Lipaseaktivität erzielt.

Es wurde bereits die Wirksamkeit einer Behandlung mit bakterieller Lipase in Form von Burlulipase für Patienten nachgewiesen, die Probleme mit der Verdauung haben, wie Patienten, die unter einer exokrinen Pankreasinsuffizienz aufgrund einer Mukoviszidose leiden. Die Verdauung wurde hierdurch deutlich verbessert, eine deutliche Verringerung von ansonsten auftretendem Fettdurchfall wurde ebenfalls beobachtet.

### Kurze Beschreibung der Zeichnungen

Nachfolgend wird die vorliegende Erfindung anhand von Figuren in Einzelheiten erläutert. Jedoch soll die Erfindung nicht hierauf beschränkt werden. In den Zeichnungen zeigt
- Figur 1: ein beispielhaftes Sondensystem in Form einer nasogastralen oder nasointestinalen Sonde im menschlichen Körper anhand einer schematischen Darstellung;
- Figur 2: eine weiteres beispielhaftes Sondensystem in Form einer PEG- bzw. PEJ-Sonde im menschlichen Körper anhand einer schematischen Darstellung;
- Figur 3: eine beispielhafte zweilumige Sonde in einer schematischen Seitenansicht und
- Figuren 4a, 4b und 4c: einen Schnitt durch die Sonde entlang der Linie 3 - 3 von Figur 3 in vergrößerten Darstellungen.

### Bevorzugte Ausführungsformen der Erfindungen

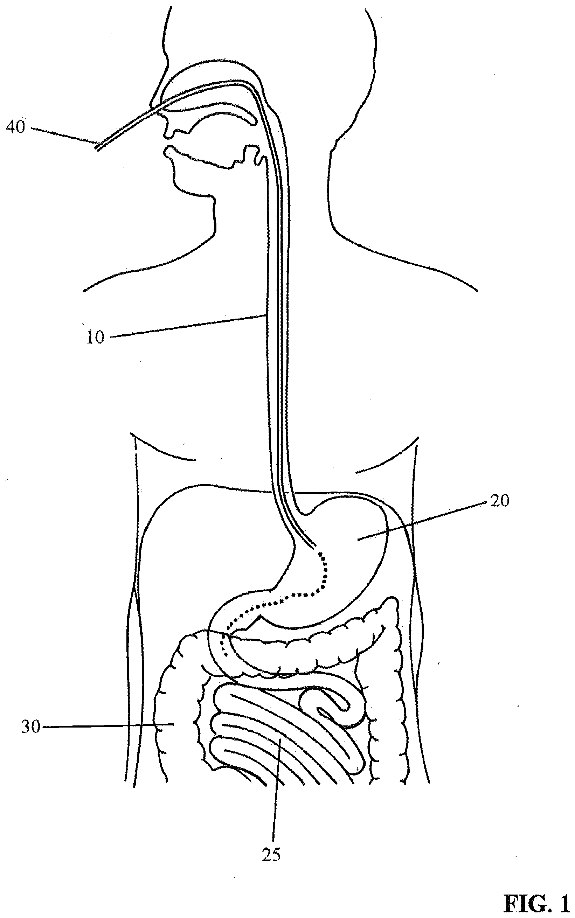

Figur 1 zeigt eine beispielhafte Ausführungsform für ein Sondensystem in Form einer nasogastralen oder nasointestinalen Sonde in einer schematischen Darstellung, veranschaulicht in einem Querschnitt des menschlichen Körpers.

Für die Verwendung wird wie folgt vorgegangen: Zunächst wird eine pharmazeutische Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase (INN) in flüssiger Darreichungsform, hergestellt. Dies kann beispielweise im Voraus industriell, aber auch in einem Krankenhaus oder einer Klinik, wo sich der Patient befindet, erfolgen. Hierzu wiederum wird eine lagerstabile, beispielsweise eine bakterielle Lipase enthaltende Arzneiform bereitgestellt, die in fester, halbfester oder bereits flüssiger Form erhältlich ist oder bereits vorliegen kann. Ganz besonders bevorzugt wird eine konzentrierte Burlulipase-Lösung verwendet. Sofern sie nicht bereits selbst die entsprechenden Kriterien erfüllt, wird diese Arzneiform dann, insbesondere durch Lösen, Dispergieren und/oder Verdünnen, in eine angemessen verdünnte flüssige Darreichungsform, die zur Verabreichung durch eine Sonde geeignet ist, überführt, beispielsweise unter Verwendung von Wasser. Eine hierfür grundsätzlich geeignete flüssige Darreichungsform ist beispielsweise eine Suspension, Emulsion oder Lösung.

Weiterhin wird eine geeignete für die künstliche Ernährung vorgesehene Sondennahrung bereitgestellt. Diese wird auf den jeweiligen Patienten, abhängig von Alter, Größe und Gewicht, Gesundheitszustand usw., abgestimmt. Zur Verwendung werden dann die pharmazeutische Zusammensetzung in Kombination mit der Sondennahrung für eine gleichzeitige, aber separat voneinander erfolgende Verabreichung formuliert. Sowohl die Sondennahrung als auch die pharmazeutische Zusammensetzung stehen daher gleichzeitig zur Verfügung, sind aber nicht miteinander in direktem Kontakt, sondern liegen getrennt voneinander vor. Beide Komponenten werden daher in Kombination bereitgestellt, separat, aber gleichzeitig verabreicht und kommen erst nach der Aufnahme durch den Patienten miteinander in Kontakt, um im Magen-Darm-Trakt die Verdauung zu bewirken.

Die flüssige Darreichungsform und die Sondennahrung werden dem Patienten dann über ein Sondensystem verabreicht. Dies wird insbesondere kontinuierlich und/oder vorzugsweise über eine mehrlumige Sonde durchgeführt. Figur 1 zeigt wie eine derartige nasogastrale oder nasointestinale Sonde 40 im menschlichen Verdauungssystem vorliegt, die je nach Länge der Sonde 40 im Magen 20 oder im Dünndarm 25 (in Figur 1 gestrichelt gezeigt) endet. Die Sonde soll im gezeigten Beispielfall eine doppellumige Sonde 40 darstellen. Es können auch andere mehrlumige Sonden als die gezeigte zum Einsatz kommen.

Über eines der Lumen der doppellumigen Sonde 40 wird die flüssige Darreichungsform, enthaltend mindestens ein Verdauungsenzym, bevorzugt einer Lipase, besonders bevorzugt eine bakterielle Lipase, ganz besonders bevorzugt Burlulipase (INN), geleitet, während die Sondennahrung parallel über das andere Lumen appliziert wird. An der Sondenmündung fließen die flüssige Darreichungsform der vorliegenden Erfindung und die Sondennahrung gleichzeitig aus den beiden Lumen direkt und gleichmäßig in den Magen bzw. Zwölffingerdarm und bieten so optimale Voraussetzungen für eine homogene Durchmischung.

Das Ende der Sonde 40 kann im Magen 20 oder im Zwölffingerdarm, d. h. im ersten Abschnitt des Dünndarms 25, liegen, wobei die Lage im Zwölffingerdarm häufig vorteilhafter ist. Flüssige Darreichungsform und Sondennahrung können gemäß einer bevorzugten Ausführungsform jeweils von einem separaten Behältnis dem jeweiligen Kanal der doppellumigen Sonde 40 zugeführt werden, aber auch aus einem Mehrkammerbehältnis mit entsprechender Zuleitung zur Sonde 40. Dabei ist die Geschwindigkeit (Volumen pro Zeit) der Applikation vorteilhafterweise durch getrennte, separat regelbare Infusions- bzw. Pumpsysteme für jeden Kanal individuell steuerbar.

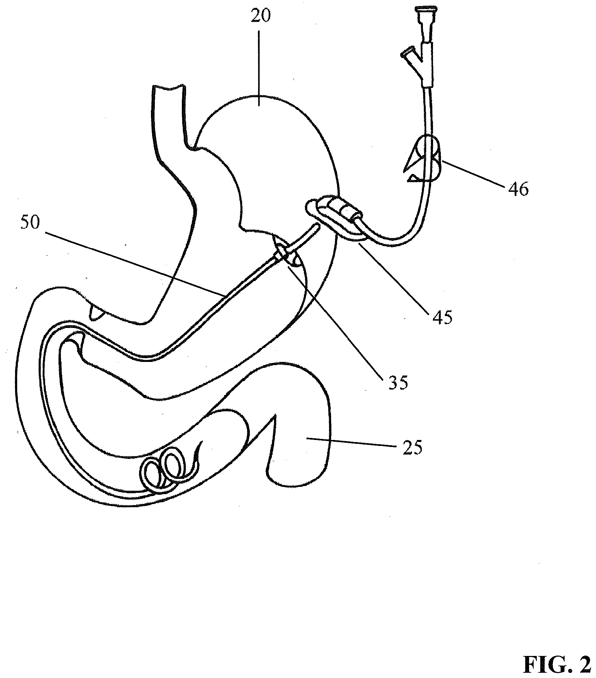

Figur 2 zeigt ein weiteres beispielhaftes Sondensystem in Form einer PEG- bzw. PEJ-Sonde anhand einer schematischen Darstellung. Hierbei wird unter endoskopischer Kontrolle die Ernährungssonde direkt in den Magen (PEG) oder in den oberen Dünndarm (PEJ) platziert. Figur 2 zeigt einen schematischen Ausschnitt des Magen-/Darm-Systems mit einer derartigen Sonde 50. Die doppellumige Sonde 50 wird im gezeigten Beispiel durch eine innere Silikon-Rückhalteplatte 35 an der Innenwand des Magens 20 fixiert. Zusätzlich wird die Sonde 50 auf der Bauchdecke durch eine äußere Halteplatte 45 und eine Schlauchklemme 46 gesichert. Auch bei der gezeigten PEJ-Sonde 50 wird die flüssige Darreichungsform, enthaltend mindestens ein Verdauungsenzym, bevorzugt eine Lipase, besonders bevorzugt eine bakterielle Lipase, ganz besonders bevorzugt Burlulipase (INN), durch eines der Lumen der Sonde appliziert, während gleichzeitig über ein anderes Lumen, getrennt hiervon, dem Patienten die Sondennahrung zugeführt wird.

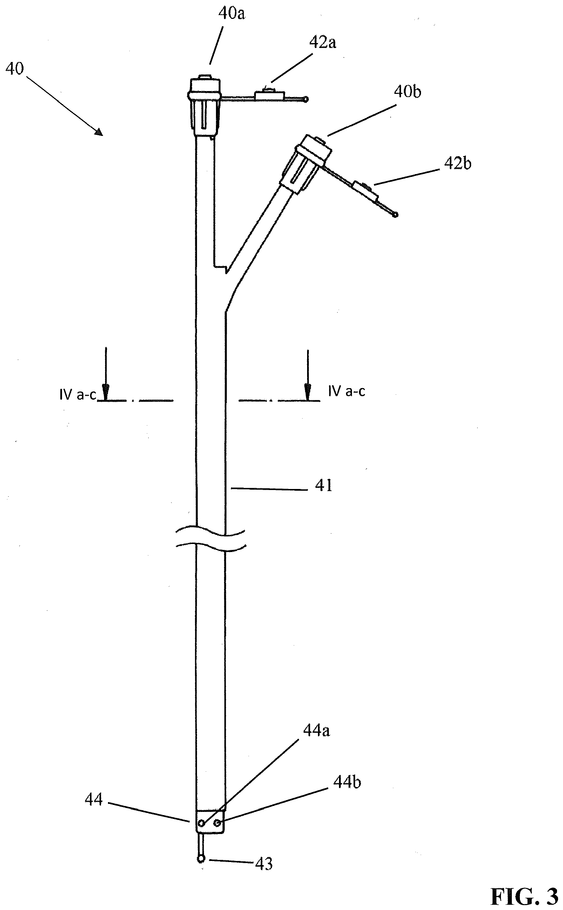

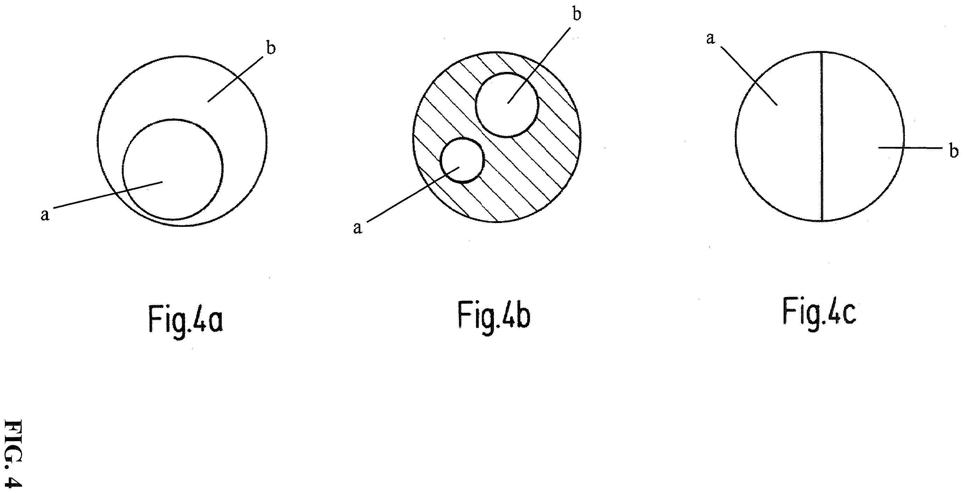

In Figur 3 ist eine beispielhafte zweilumige Sonde 40 in einer schematischen Seitenansicht gezeigt. Die Sonde 40 weist einen flexiblen Sondenschlauch 41 aus Polyurethan mit einer Länge von 100 cm auf, um in das Duodenum oder das Jejunum des Patienten eingeführt werden zu können. Die Sonde 40 ist zweilumig und weist zwei Lumina a und b auf. Das erste Lumen a kann einen identischen oder anderen Innendurchmesser als das zweite Lumen b haben. Dies ist beispielhaft in den Figuren 4a, 4b und 4c gezeigt, die einen Schnitt durch die Sonde entlang der Linie 3 - 3 von Figur 3 in vergrößertem Maßstab darstellen.

In Figur 3 weist die Sonde 40 am distalen Ende eine Olive 44 auf, die mit einem Griffansatz 43 versehen sein kann, um diesen beispielsweise mit einem Endoskop greifen zu können. Die Olive 44 weist in der Seitenwand verteilt angeordnete Öffnungen 44a und 44b auf, von denen einige im Bereich des ersten Lumens a und einige im Bereich des zweiten Lumens b angeordnet sind, so dass Flüssigkeit aus dem ersten Lumen a durch die zugeordneten Öffnungen 44a und Flüssigkeit aus dem zweiten Lumen b durch die zugeordneten Öffnungen 44b austreten kann.

Am proximalen Ende des Sondenschlauchs 41 ist ein Anschlussstück angebracht, im gezeigten Beispielfall ein positives Luer-Lock-Anschlussstück 40a mit einer Verschlusskappe 42a, das mit dem ersten Lumen a verbunden ist. Das zweite Lumen b ist mit dem weiteren Anschlussstück 40b verbunden und ebenfalls mit einer Verschlusskappe 42b versehen. Andere Ausführungen sind ebenfalls möglich.

Beispielsweise kann so mit der Sonde über das erste Lumen a die flüssige pharmazeutische Zusammensetzung verabreicht werden und über das zweite Lumen b die Sondennahrung. Beide Flüssigkeiten vermischen sich daher erst nach Austritt aus der Sonde 40 durch die Öffnungen 44a und 44b, ohne vorher miteinander in Kontakt zu kommen.

Es ist daher nachvollziehbar, dass Patienten, die künstlich ernährt werden müssen, vorteilhafterweise durch die zusätzliche gleichzeitige Verabreichung der pharmazeutischen Zusammensetzung, vorzugsweise enthaltend Burlulipase, in flüssiger Darreichungsform, eine deutlich bessere Verdauung haben werden. Hierdurch können bei Patienten zusätzliche Komplikationen durch Verdauungsprobleme, wie Durchfall, vermieden werden.

### Bezugszeichenliste

- 10: Speiseröhre
- 20: Magen
- 25: Dünndarm
- 30: Dickdarm
- 35: Silikon-Rückhalteplatte
- 40: doppellumige Sonde
- 41: Sondenschlauch
- 40a,b: Anschlussstück
- 42a,b: Verschlusskappe
- 43: Griffansatz
- 44: Olive
- 44a,b: Öffnungen
- a: erstes Lumen
- b: zweites Lumen
- 45: äußere Halteplatte
- 46: Schlauchklemme
- 50: doppellumige PEG-Sonde

## Patentansprüche

1. Pharmazeutische Zusammensetzung zur Verwendung bei der Behandlung von Verdauungsstörungen, wobei die pharmazeutische Zusammensetzung in Kombination mit einer für die künstliche Ernährung vorgesehenen Sondennahrung in der Behandlung von Verdauungsstörungen bei der künstlichen Ernährung verwendet wird, wobei die pharmazeutische Zusammensetzung mindestens ein Verdauungsenzym, bevorzugt eine Lipase, besonders bevorzugt eine bakterielle Lipase, ganz besonders bevorzugt Burlulipase, in flüssiger Darreichungsform enthält oder hieraus besteht, wobei die pharmazeutische Zusammensetzung und die Sondennahrung gleichzeitig, aber separat voneinander verabreicht werden.

2. Pharmazeutische Zusammensetzung zur Verwendung bei der Behandlung von Verdauungsstörungen nach Anspruch 1, **dadurch gekennzeichnet, dass** die pharmazeutische Zusammensetzung mindestens eine bakterielle Lipase enthält oder hieraus besteht.

3. Pharmazeutische Zusammensetzung zur Verwendung bei der Behandlung von Verdauungsstörungen nach Anspruch 1 oder 2, **dadurch gekennzeichnet, dass** die pharmazeutische Zusammensetzung Burlulipase in einer flüssigen Darreichungsform enthält oder hieraus besteht.

4. Pharmazeutische Zusammensetzung zur Verwendung bei der Behandlung von Verdauungsstörungen nach einem der vorangehenden Ansprüche 1 bis 3, **dadurch gekennzeichnet, dass** die pharmazeutische Zusammensetzung und die Sondennahrung für eine gleichzeitige, aber separat voneinander erfolgende Verabreichung derart formuliert sind, dass diese mittels eines zumindest doppellumigen Sondensystems zugeführt werden können, wobei die Sondennahrung einem Lumen des Sondensystems und die pharmazeutische Zusammensetzung in flüssiger Darreichungsform einem anderen Lumen des Sondensystems zugeführt werden.

5. Pharmazeutische Zusammensetzung zur Verwendung bei der Behandlung von Verdauungsstörungen nach einem der vorangehenden Ansprüche 1 bis 4, **dadurch gekennzeichnet, dass** die flüssige Darreichungsform eine Lösung, eine Suspension oder eine Emulsion darstellt.

6. Pharmazeutische Zusammensetzung zur Verwendung bei der Behandlung von Verdauungsstörungen nach einem der vorangehenden Ansprüche 1 bis 5, **dadurch gekennzeichnet, dass** das Sondensystem in Form einer Sonde ausgewählt wird, die im Magen, im Zwölffingerdarm oder im Leerdarm endet.

7. Pharmazeutische Zusammensetzung zur Verwendung bei der Behandlung von Verdauungsstörungen nach einem der vorangehenden Ansprüche 1 bis 6, **dadurch gekennzeichnet, dass** das Sondensystem in Form einer Sonde ausgewählt wird, die eine Nasogastral-Sonde, Nasointestinal-Sonde, Nasoduodenal-Sonde, Nasojejunal-Sonde, PEJ-Sonde oder eine PEG-Sonde darstellt und/oder dass das Sondensystem zwei- oder mehrlumig ausgeführt ist.

8. Pharmazeutische Zusammensetzung zur Verwendung bei der Behandlung von Verdauungsstörungen nach einem der vorangehenden Ansprüche 1 bis 7, **dadurch gekennzeichnet, dass** die pharmazeutische Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase in flüssiger Darreichungsform, und die Sondennahrung jeweils von einem separaten Behältnis dem jeweiligen Lumen des zumindest doppellumigen Sondensystems zugeführt werden.

9. Pharmazeutische Zusammensetzung zur Verwendung bei der Behandlung von Verdauungsstörungen nach einem der vorangehenden Ansprüche 1 bis 8, **dadurch gekennzeichnet, dass** die pharmazeutische Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase in flüssiger Darreichungsform, und die Sondennahrung in einem Mehrkammerbehältnis in getrennten Kammern bereitgestellt werden und durch getrennte Zuleitungen zum jeweiligen Lumen des zumindest doppellumigen Sondensystems zugeführt werden.

10. Pharmazeutische Zusammensetzung zur Verwendung bei der Behandlung von Verdauungsstörungen nach einem der vorangehenden Ansprüche 1 bis 9, **dadurch gekennzeichnet, dass** die Geschwindigkeit (Volumen pro Zeit) der Verabreichung der pharmazeutischen Zusammensetzung, enthaltend oder bestehend aus mindestens einem Verdauungsenzym, bevorzugt einer Lipase, besonders bevorzugt einer bakteriellen Lipase, ganz besonders bevorzugt Burlulipase in flüssiger Darreichungsform, und der Sondennahrung durch getrennte, separat regelbare Infusions- oder Pumpsysteme für jedes Lumen des zumindest doppellumigen Sondensystems einzeln steuerbar ist.

11. Pharmazeutische Zusammensetzung zur Verwendung bei der Behandlung von Verdauungsstörungen nach einem der vorangehenden Ansprüche 1 bis 10, **dadurch gekennzeichnet, dass** die pharmazeutische Zusammensetzung ein oder mehrere Lösungsmittel und/oder einen oder mehrere Hilfsstoffe aufweist, vorzugsweise ausgewählt aus Wasser, Salzen, organischen Säuren, Aminosäuren, Detergentien, Zuckern, Ölen und/oder Viskositätsregulierungsmitteln.

12. Pharmazeutische Zusammensetzung zur Verwendung bei der Behandlung von Verdauungsstörungen nach einem der vorangehenden Ansprüche 1 bis 11, **dadurch gekennzeichnet, dass** die bakterielle Lipase in Form von Burlulipase, in der pharmazeutischen Zusammensetzung in flüssiger Darreichungsform in einer Konzentration von 0,0002 mg/ml bis 50 mg/ml, bezogen auf das Burlulipase-Protein, vorliegt, bevorzugt in einer Konzentration von 0,002 mg/ml bis 5 mg/ml, bezogen auf das Burlulipase Protein, vorliegt und besonders bevorzugt in einer Konzentration von 0,01 mg/ml bis 2 mg/ml, bezogen auf das Burlulipase-Protein, vorliegt.

## Claims

1. Pharmaceutical composition for use in the treatment of digestive disorders, wherein the pharmaceutical composition is used in combination with a tube food provided for artificial nutrition in the treatment of digestive disorders in artificial nutrition, wherein the pharmaceutical composition contains or consists of at least one digestive enzyme, preferably a lipase, particularly preferably a bacterial lipase, very particularly preferably burlulipase in liquid dosage form, wherein the pharmaceutical composition and the tube food is being administered simultaneously but separately from one another.

2. Pharmaceutical composition for use in the treatment of digestive disorders according to claim 1, **characterized in that** the pharmaceutical composition contains or consists of at least a bacterial lipase.

3. Pharmaceutical composition for use in the treatment of digestive disorders according to claim 1 or 2, **characterized in that** the pharmaceutical composition contains or consists of burlulipase in a liquid dosage form.

4. Pharmaceutical composition for use in the treatment of digestive disorders according to one of the preceding claims 1 to 3, **characterized in that** the pharmaceutical composition and the tube food are formulated for simultaneous but separate administration in such a way that they can be supplied by means of an at least double-lumen tube system, wherein the tube food is supplied to one lumen of the tube system and the pharmaceutical composition is supplied in liquid dosage form to another lumen of the tube system.

5. Pharmaceutical composition for use in the treatment of digestive disorders according to one of the preceding claims 1 to 4, **characterized in that** the liquid dosage form is a solution, a suspension or an emulsion.

6. Pharmaceutical composition for use in the treatment of digestive disorders according to one of the preceding claims 1 to 5, **characterized in that** the tube system is selected in the form of a tube which ends in the stomach, in the duodenum or in the jejunum.

7. Pharmaceutical composition for use in the treatment of digestive disorders according to one of the preceding claims 1 to 6, **characterized in that** the tube system is selected in the form of a tube which is a nasogastric tube, a nasointestinal tube, a nasoduodenal tube, a nasojejunal tube, a PEJ tube or a PEG tube and / or that the tube system is designed with two or more lumens.

8. Pharmaceutical composition for use in the treatment of digestive disorders according to one of the preceding claims 1 to 7, **characterized in that** the pharmaceutical composition containing or consisting of at least one digestive enzyme, preferably a lipase, particularly preferably a bacterial lipase, very particularly preferably burlulipase in liquid dosage form, and the tube food are each supplied from a separate container to the respective lumen of the at least double-lumen tube system.

9. Pharmaceutical composition for use in the treatment of digestive disorders according to one of the preceding claims 1 to 8, **characterized in that** the pharmaceutical composition containing or consisting of at least one digestive enzyme, preferably a lipase, particularly preferably a bacterial lipase, very particularly preferably burlulipase in liquid dosage form, and the tube food are provided in separate chambers in a multi-chamber container and are supplied through separate supply lines to the respective lumen of the at least double-lumen tube system.

10. Pharmaceutical composition for use in the treatment of digestive disorders according to one of the preceding claims 1 to 9, **characterized in that** the rate (volume per time) of the administration of the pharmaceutical composition, containing or consisting of at least one digestive enzyme, preferably a lipase, particularly preferably a bacterial lipase, very particularly preferably burlulipase in liquid dosage form, and the tube food can be controlled individually by separate, individually controllable infusion or pump systems for each lumen of the at least double-lumen tube system.

11. Pharmaceutical composition for use in the treatment of digestive disorders according to one of the preceding claims 1 to 10, **characterized in that** the pharmaceutical composition has one or more solvents and/or one or more excipients, preferably selected from water, salts, organic acids, amino acids, detergents, sugars, oils and/or viscosity regulators.

12. Pharmaceutical composition for use in the treatment of digestive disorders according to one of the preceding claims 1 to 11, **characterized in that** the bacterial lipase is present in the form of burlulipase, in the pharmaceutical composition in liquid dosage form in a concentration of 0.0002 mg/ml to 50 mg/ml, based on the burlulipase protein, preferably is present in a concentration of 0.002 mg/ml to 5 mg/ml, based on the burlulipase protein, and particularly preferably is present in a concentration of 0.01 mg/ml to 2 mg/ml, based on the burlulipase protein.

## Revendications

1. Composition pharmaceutique destinée à être utilisée dans le traitement des troubles digestifs, dans laquelle la composition pharmaceutique est employée lors de l'alimentation artificielle en combinaison avec une alimentation par sonde prévue pour l'alimentation artificielle dans le traitement des troubles digestifs, dans laquelle la composition pharmaceutique contient au moins une enzyme de digestion, de préférence une lipase, particulièrement de préférence une lipase bactérienne, tout particulièrement de préférence une burlulipase, présentée sous forme liquide, ou est composée à partir de celle-ci, dans laquelle la composition pharmaceutique et l'alimentation par sonde sont administrées parallèlement mais séparément l'une de l'autre.

2. Composition pharmaceutique destinée à être utilisée dans le traitement des troubles digestifs selon la revendication 1, **caractérisée en ce que** la composition pharmaceutique contient au moins une lipase bactérienne ou est composée à partir de celle-ci.

3. Composition pharmaceutique destinée à être utilisée dans le traitement des troubles digestifs selon la revendication 1 ou 2, **caractérisée en ce que** la composition pharmaceutique contient au moins une burlulipase présentée sous forme liquide ou est composée à partir de celle-ci.

4. Composition pharmaceutique destinée à être utilisée dans le traitement des troubles digestifs selon l'une des revendications précédentes 1 à 3, **caractérisée en ce que** la composition pharmaceutique et l'alimentation par sonde sont ainsi formulées pour une administration se produisant simultanément mais séparément l'une de l'autre de telle façon que celles-ci peuvent être alimentées au moyen d'un système de sonde à au moins double lumière, dans laquelle l'alimentation par sonde est amenée à une lumière du système de sonde et la composition pharmaceutique présentée sous forme liquide est amenée à une autre lumière du système de sonde.

5. Composition pharmaceutique destinée à être utilisée dans le traitement des troubles digestifs selon l'une des revendications précédentes 1 à 4, **caractérisée en ce que** la forme d'administration liquide est une solution, une suspension ou une émulsion.

6. Composition pharmaceutique destinée à être utilisée dans le traitement des troubles digestifs selon l'une des revendications précédentes 1 à 5, **caractérisée en ce que** le système de sonde est sélectionné sous forme d'une sonde qui finit dans l'estomac, le duodénum ou le jéjunum.

7. Composition pharmaceutique destinée à être utilisée dans le traitement des troubles digestifs selon l'une des revendications précédentes 1 à 6, **caractérisée en ce que** le système de sonde est sélectionné sous forme d'une sonde qui est une sonde gastro-nasale, une sonde naso-intestinale, une sonde naso-duodénale, une sonde naso-jéjunale, une sonde PEJ ou une sonde PEG et/ou que le système de sonde est conçu à deux lumières ou plus.

8. Composition pharmaceutique destinée à être utilisée dans le traitement des troubles digestifs selon l'une des revendications précédentes 1 à 7, **caractérisée en ce que** la composition pharmaceutique, comprenant ou se composant d'au moins une enzyme de digestion, de préférence une lipase, particulièrement de préférence une lipase bactérienne, tout particulièrement de préférence une burlulipase présentée sous forme liquide, et l'alimentation par sonde sont amenées respectivement depuis un récipient séparé, vers la lumière respective du système de sonde à au moins deux lumières.

9. Composition pharmaceutique destinée à être utilisée dans le traitement des troubles digestifs selon l'une des revendications précédentes 1 à 8, **caractérisée en ce que** la composition pharmaceutique, comprenant ou se composant d'au moins une enzyme de digestion, de préférence une lipase, particulièrement de préférence une lipase bactérienne, tout particulièrement de préférence une burlulipase présentée sous forme liquide, et l'alimentation par sonde sont fournies dans des chambres séparées dans un récipient à plusieurs chambres et amenées par des tuyaux séparés vers la lumière respective du système de sonde à au moins deux lumières.

10. Composition pharmaceutique destinée à être utilisée dans le traitement des troubles digestifs selon l'une des revendications précédentes 1 à 9, **caractérisée en ce que** la vitesse (volume/temps) d'administration de la composition pharmaceutique, comprenant ou se composant d'au moins une enzyme de digestion, de préférence une lipase, particulièrement de préférence une lipase bactérienne, tout particulièrement de préférence une burlulipase présentée sous forme liquide, et d'alimentation par sonde peut être commandée individuellement par des systèmes de perfusion ou de pompe séparés, pouvant être régulés séparément, pour chaque lumière du système de sonde à au moins deux lumières.

11. Composition pharmaceutique destinée à être utilisée dans le traitement des troubles digestifs selon l'une des revendications précédentes 1 à 10, **caractérisée en ce que** la composition pharmaceutique présente un ou plusieurs diluant(s) et/ou un ou plusieurs excipient(s), de préférence sélectionnés parmi l'eau, des sels, des acides organiques, des amino-acides, des détergents, des sucres, des huiles et/ou des moyens de régulation de la viscosité.

12. Composition pharmaceutique destinée à être utilisée dans le traitement des troubles digestifs selon l'une des revendications précédentes 1 à 11, **caractérisée en ce que** la lipase bactérienne sous forme de burlulipase, est présente dans la composition pharmaceutique présentée sous forme liquide dans une concentration de 0,0002 mg/ml à 50 mg/ml, rapporté à la protéine de burlulipase, de préférence dans une concentration de 0,002 mg/ml à 5 mg/ml, rapporté à la protéine de burlulipase, et particulièrement de préférence dans une concentration de 0,01 mg/ml à 2 mg/ml, rapporté à la protéine de burlulipase.
